(19) Europäisches Patentamt
European Patent Office
Office européen des brevets

(11) EP 3 251 690 B1

(12) **EUROPEAN PATENT SPECIFICATION**

(45) Date of publication and mention of the grant of the patent:
**06.08.2025 Bulletin 2025/32**

(21) Application number: **17173678.8**

(22) Date of filing: **31.05.2017**

(51) International Patent Classification (IPC):
***A61K 39/00*** *(2006.01)* ***A61K 39/385*** *(2006.01)*
***G01N 33/487*** *(2006.01)* ***G01N 33/94*** *(2006.01)*

(52) Cooperative Patent Classification (CPC):
**G01N 33/9486; A61K 39/0013; A61K 39/385; C07D 295/096; C07D 333/32; C12N 9/0065; C12Y 111/01007;** A61K 2039/6031; A61K 2039/6081

(54) **MT-45 IMMUNODETECTION**

MT-45 IMMUNODETEKTION

IMMUNODETECTION DE MT-45

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.05.2016 GB 201609473**

(43) Date of publication of application:
**06.12.2017 Bulletin 2017/49**

(73) Proprietor: **Randox Laboratories Ltd.**
**Crumlin Antrim BT29 4QY (GB)**

(72) Inventors:
- **Benchikh, Elouard**
  **Crumlin, Antrim BT29 4QY (GB)**
- **Fitzgerald, Peter**
  **Crumlin, Antrim BT29 4QY (GB)**
- **Lowry, Philip**
  **Crumlin, Antrim BT29 4QY (GB)**
- **McConnell, Ivan**
  **Crumlin, Antrim BT29 4QY (GB)**

(56) References cited:
**EP-A1- 2 679 241**

- **PAPSUN DONNA ET AL: "Analysis of MT-45, a Novel Synthetic Opioid, in Human Whole Blood by LC-MS-MS and its Identification in a Drug-Related Death.", JOURNAL OF ANALYTICAL TOXICOLOGY MAY 2016, vol. 40, no. 4, 1 May 2016 (2016-05-01), pages 313 - 317, XP002774046, ISSN: 1945-2403**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 251 690 B1

## Description

### BACKGROUND

MT-45, systematic name (*R,S*)-1-cyclohexyl-4-(1,2-diphenylethyl)piperazine, is a synthetic drug of abuse of the opioid class, the S-enantiomer having high affinity for delta and kappa opioid receptors. It has therapeutic origins and was a candidate analgesic drug having a potency similar to morphine but has since been detected in several biological samples of individuals subject to fatal and non-fatal intoxication (reference 1). Given the emergence of this toxic drug of abuse and its on-going legislative control, methods for its detection in biological and pre-ingested samples are required. Gas chromatography (GC) and Liquid chromatography (LC) linked to mass spectrometry (MS), Fourier Transform infrared spectroscopy and proton and carbon-13 nuclear magnetic resonance spectroscopy have been described for the detection of MT-45 (reference 2). Papsun (2016) describes detection and quantification of MT-45 in blood samples using LC-MS-MS. These techniques require specialist staff for their operation, are expensive and are not amenable to use outside of the laboratory.

*References*

1. European Monitoring Centre for Drugs and Drug Addiction (2014), EMCDDA-Europol Joint Report on a new psychoactive substance: 1-cyclohexyl-4-(1,2-diphenylethyl)piperazine ('MT-45'), Joint Reports, Publications Office of the European Union, Luxembourg.
2. European Monitoring Centre for Drugs and Drug Addiction (2014), Risk Assessment Report of a new psychoactive substance: 1-cyclohexyl-4-(1,2-diphenylethyl)piperazine (MT-45).
3. Papsun et al (2016). Analysis of MT-45, a novel synthetic opioid, in human whole blood by LC-MS-MS and its identification in a drug-related death. Journal of Analytical Toxicology, 40:313-317.

### SUMMARY OF THE INVENTION

Described are the first known immunogens and antibodies for MT-45. The invention further describes detecting agents and methods and kits comprising the antibody.

### Figures

Figure 1 MT-45 hapten A and hapten B
Figure 2 Synthesis of hapten A
Figure 3 Synthesis of (*R*,*S*)-1-cyclohexyl-4-[2-(4-carboxymethoxyphenyl)-1-phenylethyl]piperazine hapten
Figure 4 Immunogens of hapten A
Figure 5 Immunogens based on derivatisation of the 4-position of the phenyl moiety attached to the **2**-ethyl position of MT-45
Figure 6 Detecting agents (tracers)

### DETAILED DESCRIPTION OF THE INVENTION

In a first aspect, the invention describes an immunogen of structure I.

phenyl ring 1

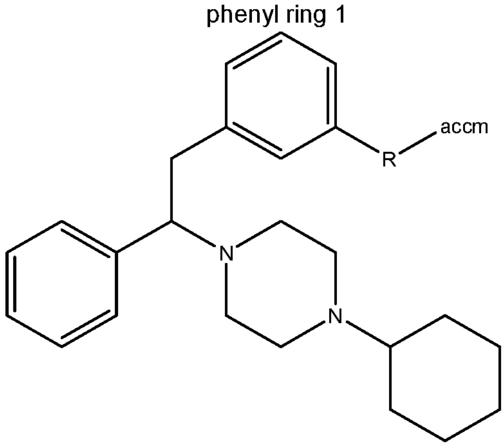

structure I

in which R is a spacing group attached to the meta position of the phenyl ring (phenyl ring 1) and the accm is an antigenicity conferring carrier material. The spacing group (also known as a crosslinker or crosslinking group) is standard in the art and provides a means to both link and impart a degree of separation between the small molecule (in this case MT-45) to which antibodies are to be raised and the accm. For example, it can be a short chain substituted or unsubstituted alkanediyl chain usually supporting a functional group (e.g. carboxy, amino, carbonyl, ester) at one or both of the chain or ring end(s), following or preceding attachment to the phenyl ring and the accm i.e. the spacing group is made up of atoms that can be already part of MT-45 or a MT-45 analogue, or can be added to MT-45 or a MT-45 analogue through chemical synthesis, or can be a combination of both e.g. see Figures 2 & 3 and Examples 5 and 6 in which an MT-45 analogue is produced incorporating an oxygen atom (part of spacing group) on phenyl ring 1 during its synthesis prior to addition of a carboxymethylene moiety (part of spacing group). The total linear chain length of the crosslinker is preferably 1-10 atoms, most preferably 1-6 atoms. For the invention, R is -(Q)m-Y-X-, and m= 0 or 1; Y is a $C_{1-6}$ substituted or unsubstituted, straight chain alkanediyl; X, before attachment to the antigenicity conferring carrier material, is chosen from carboxy ($-CO_2H$), dithiopyridyl, maleimidyl, amino ($-NH_2$), hydroxyl (-OH), thiol (-SH), thioester (-C(O)-S-R or -C(S)-O-R, in which R =alkyl) or aldehyde (-C(O)-R, in which R is H or alkyl); Q is -O-, -S-, or -NH-. In a preferred embodiment of the invention, -(Q)m-Y-X-comprises: Y= $C_{1-6}$, substituted or unsubstituted, straight chain, alkanediyl moiety; m =1 and Q is -O-, -NH- or -S-. Antigenicity conferring carrier materials are well known and well-established in the art and can be any material that makes all or part of the hapten immunogenic, such as a protein, a protein fragment, a synthetic polypeptide or a semi-synthetic polypeptide. The most commonly used and $accm_s$ of the current invention are keyhole limpet haemocyanin (KLH), bovine thyroglobulin (BTG), bovine serum albumin (BSA), egg ovalbumin, bovine gamma globulin or cationised BSA (see General Methods, Examples and Results for further examples). The process of immunogen formation generally involves coupling of a hapten to a crosslinking agent, the latter subsequently coupled to an accm. Alternatively, the crosslinking agent can be coupled to the accm in the first instance and subsequently couples to the hapten. In both these processes the crosslinking agent generally incorporates at least two reactive functional groups. Numerous crosslinkers and accms are commercially available and have been described in the literature (Thermo Scientific Crosslinking Technical Handbook, 1606073 04/2009; Bioconjugate Techniques G.Hermanson, ed, Academic Press, 1996, 785pp).

The invention further describes an immunoassay method of detecting or determining MT-45 in an *in vitro* sample or in a solution comprising contacting the sample or solution with a detecting agent and an antibody raised from an immunogen of the invention, detecting the bound detecting agent and deducing the presence or amount of MT-45. By 'detecting' is meant qualitatively analysing for the presence or absence of a substance; by 'determining' is meant quantitatively analysing for the amount of a substance present. The detecting agent when added to a sample competes with MT-45 present in the sample for binding to the antibody. The detecting agent is of structure II

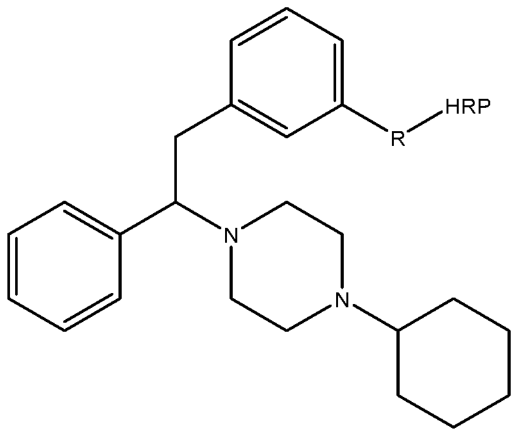

structure II

in which R is a crosslinking group attached to the meta position of the phenyl ring and HRP is horseradish peroxidase. The crosslinking group R of the detecting agent can take all the values of R described for structure III, the immunogen i.e. in which R is $-(Q)_m$-Y-X- and in which in the case of the detecting agent, X is attached to HRP. Examples of detecting agents are given in Examples 9 and 10 of the General Methods, Examples and Results section. The antibodies described herein were derived from immunogens produced from haptenic racemic mixtures; using asymmetric synthesis or enantiomeric separation, the (*S*) or (*R*) enantiomer haptens can also be used for immunogen formation and as detecting agents, and unless otherwise indicated, structures depicted herein are also meant to include, as well as racemates and enantiomers, other stereoisomeric forms such as diastereomers and cis-trans conformers. The term "hapten" refers to small molecule pre-immunogen precursors e.g. molecule 6 of Figure 2. The detection or determination step is usually qualified or quantified with the aid of a calibrator. A calibrator is well known in the art and enables a threshold concentration or the exact or calibrator equivalent amount of an analyte to be determined. A calibrator curve or threshold concentration may be suitable for several analytes or may have to be derived for each individual analyte. The determination of an exact or calibrator equivalent amount of an analyte often requires the construction of a calibration curve (also known as a standard curve). The number of calibrator points can vary, but is usually from 5 to 9. The *in vitro* sample is a biological sample such as blood, serum, plasma, urine or saliva. The *in vitro* sample is preferably a serum, plasma or urine sample. The solution can be a liquid suspected of containing MT-45. Alternatively, as MT-45 is often in solid form, analysis may require pre-treatment to achieve a formulation suitable for immuno-analysis, such as dissolution in a suitable liquid. The immunoassay method is preferably based on the well-known competitive assay format in which a target analyte which binds to the antibody i.e. the molecule to be detected or determined, competes with a detecting agent which also binds to the antibody, for binding sites on the antibody. The detecting agent can be any substance which leads to a detectable or measurable signal and typically incorporates an enzyme which promotes light emission from a substrate, a fluorophore or a radioactive label; it is usual for an immunoassay that the detecting agent incorporates a structure similar to the target analyte to which an enzyme or a substance having fluorescent properties has been conjugated, or in which a radiolabel has been incorporated. For the immunoassay method of the invention, the detecting agent is based on a compound with structure II. Conjugation of R is by way of standard methods familiar to the skilled person and may involve the crosslinking methodology and groups described previously for the immunogen of the invention.

The invention further describes an antibody raised from an immunogen of structure I. The immunogen from which the antibody is derived has the spacing group attached to the meta position of the phenyl ring of MT-45. More preferably the antibody is raised from an immunogen of structure

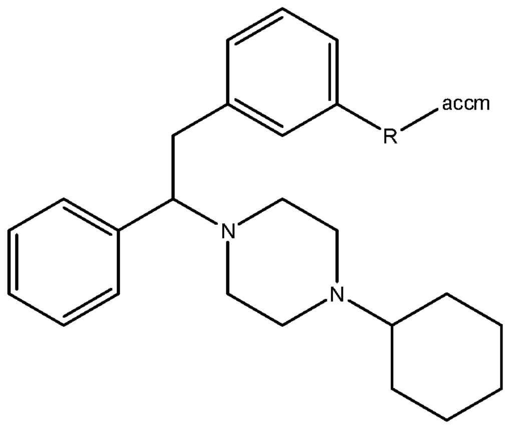
structure III

in which R is $-(Q)_m$-Y-X-, in which Y is $C_{1-6}$ substituted or unsubstituted, straight chain, alkanediyl moiety; m=1 and Q is -O-, -NH- or -S-; and X, before attachment to the accm, is chosen from carboxy, dithiopyridyl, maleimidyl, amino, hydroxyl, thiol, thioester or aldehyde. Most preferably, the antibody is derived from an immunogen in which Q is -O-, Y is $-CH_2-$ , X is carboxy and the accm is keyhole limpet haemocyanin. There are several parameters that can be used to compare the relative degree of binding of an antibody to different analytes including the lowest limit of detection, the lowest limit of quantification and the $IC_{50}$. The $IC_{50}$ is determined from a standard curve which is generated using a competitive assay (see Example 12 of the General Method, Examples and Results section and Table 1) and can be used to derive analyte cross-reactivities. To enable an assay to be effectively applied, an $IC_{50}$ of less than or about 50 ng/ml, preferably less than or about 10 ng/ml, more preferably less than or about 5 ng/ml or less than or about 1 ng/ml, for any individual analyte is preferred. Derivation of these parameters enables the relative cross-reactivities of various analytes to an antibody to be determined. In another embodiment the antibody of the invention has an $IC_{50}$ of about less than or about 20 ng/ml, more preferably less than or about 10 ng/ml, most preferably less than or about 1 ng/ml to MT-45. Preferably, the antibody is derived from an immunogen of structure III and has an $IC_{50}$ of <1.00 ng/ml to MT-45 as calculated from a standard curve using MT-45-CME-HCTL-maleimide-HRP (Tracer 2) as detecting agent. More preferably the antibody is derived from an immunogen in which Q is -O-, Y is $-CH_2-$ , X is carboxy and the accm is keyhole limpet haemocyanin and has an $IC_{50}$ of <1.00 ng/ml to MT-45 as calculated from a standard curve using MT-45-CME-HCTL-maleimide-HRP (Tracer 2) as detecting agent. Due to inter-molecular attractive forces such as hydrogen bonding and van der Waal's forces there is often a degree of binding or affinity between two molecules whatever their respective structures; the skilled person recognizes that no cross-reactivity or minimal cross-reactivity implies that in the context of a working immunoassay any binding or interaction between an antibody and non-target analytes is at such a low level that it does not compromise the integrity of the immunoassay i.e. false positives, whether qualitative or quantitative, are avoided.

The invention also describes a kit comprising an antibody of the invention and optionally a detecting agent of the invention which is preferably of structure II. Preferred kits are described in claim 7. The kit can also incorporate antibodies, derived from any of the immunogens of the invention, passively adsorbed on or chemically bonded to a solid state device. A solid state device may also be referred to as a substrate. The antibodies engage with the substrate by, for example, passive adsorption or can be chemically bonded to the substrate attached by way of, for example, covalent bonds. Such covalent bonding generally requires the initial introduction of a chemically active compound covalently attached to the substrate surface prior to antibody addition. The antibody itself may also require the addition of a chemical activating group to achieve substrate bonding. These requirements are well known in the art. The substrate can be any medium capable of adsorbing or bonding to an antibody, for example a bead, a microtitre plate or a nanoparticle (all optionally chemically-activated), but is preferably of a planar conformation (optionally chemically-activated) such as a glass slide or a biochip. A biochip or microtitre plate is the preferred substrate. A biochip is a thin, wafer-like substrate with a planar surface which can be made of any suitable material such as glass or plastic but is preferably made of ceramic. The biochip is able to be chemically-activated prior to antibody bonding or is amenable to the passive adsorption of antibodies. The skilled person in biochip development for immunoassay application will recognize that a planar surface at high resolution e.g. if using a scanning electron microscope, is not perfectly 'flat' but will possess an uneven surface, the important aspect being that the 'approximately' planar surface is suitable for application. A microlayer coating of material can optionally be added to the planar surface of the substrate prior to antibody placement. Either the upper surface or both surfaces of the substrate can be coated. The biochip can be integrated into or placed into a device with walls. Such a walled device can aid in the retention of added sample or solution. The solid state device can also support other antibodies which have a binding specificity which is different from the binding specificity of the antibodies of the invention. Such a support with multiple different antibodies is often described as a multianalyte array (reference to an 'array' includes a microarray). If the method of detection is different fluorescent labels, each different fluorescent label emitting electromagnetic radiation at a unique

wavelength, then the location of placement of the antibodies on the solid substrate is not critical. However, for antibodies forming part of a multianalyte array in which the detecting agent is, for example, a chemiluminescent molecule, the antibodies of differing specificity must not overlap and must be located in discrete areas on the solid state device. Such a system is also referred to as a spatially addressable multianalyte array.

**General Methods, Examples and Results**

*Preparation of Haptens, Immunogens and Detecting Agents*

In immunology, haptens are defined as substances which by themselves cannot elicit immune responses; they require chemical coupling to larger immunogenic molecules (antigenicity conferring carrier materials or 'accm'), to be capable of inducing an immune response. Appropriate accms commonly contain poly(amino acid) segments and include polypeptides, proteins and protein fragments. Illustrative examples of antigencity conferring carrier materials are keyhole limpet haemocyanin (KLH), bovine thyroglobulin (BTG), bovine serum albumin (BSA), egg ovalbumin, bovine gamma globulin or cationised BSA. Alternatively, synthetic poly(amino acids) having a sufficient number of available amino groups, such as lysine, may be employed, as may other synthetic or natural polymeric materials bearing reactive functional groups. Also, carbohydrates, yeasts or polysaccharides may be conjugated to the hapten to produce an immunogen. The haptens can also be coupled to a detectable labelling agent such as an enzyme (for example, horseradish peroxidase), a substance having fluorescent properties or a radioactive label for the preparation of detecting agents for use in the immunoassays. The fluorescent substance may be, for example, a monovalent residue of fluorescein or a derivative thereof. Conjugation of haptens can be performed using standard methods of conjugation such as mixed anhydride, EDC or succinimidyl activation of the haptens. In order to confirm that adequate conjugation of hapten to carrier material has been achieved, prior to immunisation, each immunogen is evaluated using matrix-assisted UV laser desorption /ionisation time-of-flight mass spectroscopy (MALDI-TOF MS).

*General Procedure for MALDI-TOF Analysis of Immunogens*

MALDI-TOF mass spectrometry was performed using a Voyager STR Biospectrometry Research Station laser-desorption mass spectrometer coupled with delayed extraction. An aliquot of each sample to be analysed was diluted in 0.1% aqueous trifluoroacetic acid (TFA) to create 1mg/ml sample solutions. Aliquots (1μl) were analysed using a matrix of sinapinic acid and bovine serum albumin (Fluka) was used as an external calibrant.

*Immunoassay Development*

The process of developing an immunoassay is well known to the person skilled in the art. A detecting agent (e.g. appropriate hapten conjugated to HRP) is added to a sample containing the target analyte and the raised antibodies, and the detecting agent and analyte compete for binding to the antibodies. The said antibodies were fixed to a polystyrene solid support (e.g. dilution of antibodies in coating buffer and incubation at 37°C for 2 hours to allow antibody binding to surface). The antibodies can be polyclonal or monoclonal using standard techniques, but the current invention makes use of polyclonal antibodies. The signal emitted in the immunoassay is proportionate to the amount of detecting agent bound to the antibodies which in turn is inversely proportionate to the analyte concentration. The signal can be detected or quantified by comparison with a calibrator with known levels of target analyte.

<u>Example 1: Synthesis of 2-(4-cyclohexylpiperazin-1-yl)-2-phenylacetonitrile</u>

<u>(Compound 2)</u>

A mixture of $H_2O$ (13ml) and sodium bisulfite (5.14g, 49.43mmol) were mixed in a beaker, with stirring. Benzaldehyde 1 (5.24g, 49.43mmol) was added and the mixture was stirred for 30mins, during which time a slurry of the benzaldehyde-bisulfite addition product formed. A 20% aq. solution of 1-cyclohexylpiperazine (8.5g, 50.42mmol) in $H_2O$ (35ml) was then added dropwise to the reaction mixture with stirring (during which most of the compound dissolved). After the addition was complete, sodium cyanide (2.42g, 49.43mmol) was added portion-wise over a period of 15mins. The reaction mixture's beaker was covered and left stirring overnight. Ethyl acetate (200ml) was added to the reaction mixture and the organic layer was separated and washed successively with water (150ml) and brine (150ml) (aqueous layers were combined and left stirring overnight with excess sodium hypochlorite to quench the remaining cyanide).The organic portion was dried over $Na_2SO_4$, filtered and concentrated to dryness in vacuo. The crude product obtained was purified by chromatography (Biotage, 80g ZIP sphere cartridge, ethyl acetate (0→50%) in pet. ether to afford Compound 2. (10.25g, 36.17mmol, 73% yield) as a white solid.

Example 2: Synthesis of 1-cyclohexyl-4-(2-(3-methoxyphenyl)-1-phenylethyl)-piperazine (3-OMe MT-45) (Compound 3).

In a flame dried 3-neck round bottom flask, with a condenser and a dropping funnel attached, under nitrogen, was added Magnesium (Mg) (5.27g, 217mmol), anhydrous diethyl ether (45ml) and a crystal of Iodine. Under vigorous stirring, at room temperature, 20-30 drops of 3-methoxybenzyl bromide (total amount needed for the reaction: 14.54g, 72.33mmol) were added to the reaction mixture. After 1-2 minutes the iodine colour fades out and the reaction mixture starts to self-reflux. Then, through the dropping funnel, the rest of the 3-methoxybenzyl bromide, dissolved in anhydrous diethyl ether (45ml), was added dropwise to the reaction mixture at such a rate that the self-reflux is maintained. After the addition completed, the flask was heated at reflux for 1h. Then, the reaction mixture was cooled down to RT and 2-(4-cyclohexylpiperazin-1-yl)-2-phenylacetonitrile 2 (10.25g, 36.17mmol) dissolved in anhydrous diethyl ether (45ml) was added dropwise. When the addition was completed, the reaction mixture was heated to reflux overnight. The reaction mixture was then cooled to RT and a saturated aq. solution of ammonium chloride (~200ml) was cautiously added and stirred for 1h. The mixture was extracted with ethyl acetate (2x250ml). The combined organic extracts were dried over Na2SO4, filtered and concentrated in vacuo to dryness. The crude product obtained was purified by chromatography (Biotage, 80g ZIP sphere cartridge, ethyl acetate (0→80%) in pet. ether) (Caution: The product has a low UV activity) to afford Compound 3 (13.5g, 35.67mmol, 98% yield).

Example 3: Synthesis of 3-(2-(4-cyclohexylpiperazin-1-yl)-2-phenylethyl) phenol (3-Hydroxy MT-45) (Compound 4).

1-cyclohexyl-4-(2-(3-methoxyphenyl)-1-phenylethyl)piperazine (13.5g, 35.67mmol) (compound 3) was dissolved in acetic acid (100ml) and HBr 48% (400ml), in a round bottom flask, and the mixture was refluxed overnight. The next morning the solution had turned black. The reaction mixture was poured into ice (300g) and, carefully, ammonium hydroxide, 28-30% solution, was added until alkaline (pH=10). Then, DCM (1L was added and the organic layer was separated, washed by brine (250ml), dried over Na2SO4, filtered and concentrated in vacuo to dryness, affording Compound 4 as a brownish solid (8.0g, 21.94mmol, yield=62%), which was used crude in Example-4.

Example 4: Synthesis of tert-butyl 2-(3-(2-(4-cyclohexylpiperazin-1-yl)-2-phenylethyl)phenoxy) acetate (Compound 5).

3-(2-(4-cyclohexylpiperazin-1-yl)-2-phenylethyl) phenol (compound 4) (3.0g, 8.24mmol) was dissolved in anhydrous DMF (40ml) under nitrogen and sodium hydride (60% suspension in mineral oils) (495mg, 12.36mmol) was added at 0oC. After 1h of stirring in the ice bath (gas evolution had ceased), t-butyl bromoacetate (1.46ml, 9.89mmol) was added and the reaction mixture was kept at 0oC for 30 mins and allowed to warm to RT. After stirring for 1h at RT, a few drops of water were carefully added to quench the excess of NaH. The volatiles were removed in vacuo and ethyl acetate (200ml) and saturated aqueous solution of NH4Cl (150ml) were added to the crude residue. The organic layer was separated, dried over Na2SO4, filtered and concentrated in vacuo. The crude product obtained was purified by chromatography (Biotage, 45g ZIP sphere cartridge, EtOAc (0→70%) in pet. ether) to afford the ester (Compound 5) (2.8g, 5.86mmol, 71% yield).

Example 5: Synthesis of 2-(3-(2-(4-cyclohexylpiperazin-1-yl)-2-phenylethyl) phenoxy)acetic acid (MT-45-3-CME, Compound 6 Hapten A).

Tert-butyl 2-(3-(2-(4-cyclohexylpiperazin-1-yl)-2-phenylethyl) phenoxy) acetate (2.8g, 5.86mmol) was mixed with DCM (40ml) and TFA (20ml). The mixture was stirred at RT overnight. The volatiles were removed in vacuo to dryness and the crude product obtained was purified by chromatography (Biotage,45g ZIP sphere cartridge, MeOH (0→30%) in ethyl acetate) to afford Compound 6 (1.50g, 3.55mmol, 61% yield) as a white solid.

Example 6: Synthesis of 3-[2-(4-cyclohexylpiperazin-1-yl)-2-phenylethyl)] phenoxy-N-(2-oxotetrahydrothiophen-3-yl) acetamide (compound 7 Hapten B).

2-(3-(2-(4-Cyclohexylpiperazin-1-yl)-2-phenylethyl) phenoxy) acetic acid (500mg, 1.18mmol) was dissolved in pyridine (16ml). DL-homocysteine thiolactone hydrochloride (273mg, 1.77mmol) was added, followed by EDCI.HCl (341mg, 1.77mmol). The reaction mixture was left to stir overnight at RT. The volatiles were removed in vacuo and the crude product obtained was purified by chromatography (Biotage via a 30g ZIP sphere cartridge, MeOH (0→20%) in ethyl acetate) to afford the titled product (420mg, 0.81mmol, 68% yield) as an orange solid.

Example 7: Conjugation of 2-(3-(2-(4-cyclohexylpiperazin-1-yl)-2-phenylethyl) phenoxy) acetic acid (MT-45-3-CME (Compound 6) (Hapten A).to BSA (Immunogen 1)

To a solution of MT-45-3-CME (Hapten A) (32.5mg) in DMF (1.0ml.) was added N-(3-dimethylaminopropyl)-N'-ethylcarbodiimide hydrochloride (EDC. HCl) (73.8mg) and N-hydroxysuccinimide (44.2mg) and the mixture was incubated on the roller at room temperature overnight. This solution was added dropwise to a solution of BSA (100mg, 1.5micomol) in phosphate buffer saline (50mM) (pH 8.0) (10ml). The resulting solution was incubated on the roller at room temperature overnight. Excess hapten was removed by dialysis against phosphate buffer saline, pH 7.2 (3 changes) for 24 hours at 4°C, and freeze-dried. MALDI results showed 26.5 molecules of MT-45-3-CME (Hapten A) had been conjugated to one molecule of BSA.

Example 8: Conjugation of 2-(3-(2-(4-cyclohexylpiperazin-1-yl)-2-phenylethyl)phenoxy)acetic acid (MT-45-3-CME (Compound 6) (Hapten A). to KLH (Immunogen 2)

To a solution of MT-45-3-CME (Hapten A) (32.5mg) in DMF (1.0ml.) was added N-(3-dimethylaminopropyl)-N'-ethylcarbodiimide hydrochloride EDC.HCl (73.7mg) and N-hydroxysuccinimide (44.3mg) and the mixture was incubated on the roller at room temperature overnight. This solution was added dropwise to a solution of KLH (100mg) in phosphate buffer saline (50mM) (pH 8.0) (10ml.). The resulting solution was incubated on the roller at room temperature overnight. Excess hapten was removed by dialysis against phosphate buffer saline, pH 7.2 (3 changes) for 24 hours at 4°C, and freeze-dried.

Example 9: Conjugation of 2-(3-(2-(4-cyclohexylpiperazin-1-yl)-2-phenylethyl) phenoxy) acetic acid (MT-45-3-CME, Compound 6 Hapten A) to HRP (Tracer 1)

EDC hydrochloride (1.5mg) was dissolved in water (0.5ml.) and immediately added to a solution of MT-45-3-CME (Hapten A) (3.0mg) in DMF (0.2ml.). After mixing, this solution was added dropwise to a solution of HRP (20mg) in water (1ml.). N-hydroxysuccinimide (1mg) was added and the reaction mixture was incubated in the dark at room temperature overnight. Excess hapten was removed with double PD-10 columns (Pharmacia) in series, pre-equilibrated with PBS at pH 7.2. The MT-45-3-CME-HRP conjugate (tracer 1) was then dialysed overnight against 10L of PBS at pH 7.2 at 4°C.

Example 10: Conjugation of 2-(3-(2-(4-cyclohexylpiperazin-1-yl)-2-phenylethyl) phenoxy)-N-(2-oxotetrahydrothiophen-3-yl) acetamide (MT-45-3-CME-HCTL) (compound 7) (Hapten B) to HRP-maleimide.(Tracer 2)

*Preparation of HRP-maleimide:* 4-(N-maleimidomethylcyclohexyl)-1-carboxylic acid NHS ester (0.84mg) in DMF (0.045ml) was added drop-wise to the HRP (20mg) dissolved in 50mM HEPES solution, pH8.5 (0.8ml) while stirring. The resulting solution was stirred at 15-25 °C for 40 minutes. Excess maleimide linker was removed by dialysis against phosphate buffer saline pH7.2. *Pre-activation of the hapten*: MT-45 3-CME HCTL (Hapten-B) (2mg) was dissolved in DMF (0.2ml). To this solution was added potassium hydroxide (1M) (0.2ml) and the solution was incubated for 10 minutes. 0.5ml of 0.2M Phosphate buffer, Ph7.0 was added to quench reaction and 0.1M HCl solution added to neutralise the solution. *Conjugation*: The HRP-maleimide was added to the activated hapten, rolled for two hours at room temperature and then cooled at 2 to 4°C and rolled overnight. Excess hapten was removed with PD-10 column (Pharmacia), pre-equilibrated with PBS pH 7.2 and dialysed at 2-8°C against PBS pH 7.2 to afford MT-45-CME-HCTL-maleimide-HRP (Tracer 2).

Example 11: Preparation of antisera

Pre-immunization blood samples were collected from young adult, female, Texel sheep. In order to generate polyclonal antisera, 2mgs of the immunogen (immunogens 1 or 2) was prepared in PBS, mixed at a ratio of 50% immunogen in PBS to 50% Freund's Complete adjuvant (Sigma, Product Number F5881) and emulsified by repeatedly passing the mixture through a tip on the end of a 1ml syringe, until the required semi-solid consistency was acquired. 1ml of the emulsified mixture was injected intramuscularly into each host animal (sheep) as the primary immunisation dose. Further injections (boosts) were prepared (1mg of immunogen in PBS and mixed at a ratio of 50% Immunogen in PBS/50% Freund's Incomplete adjuvant, Sigma, Product Number - F5506). Boost injections were delivered intramuscularly at monthly intervals, 1ml per animal. Serum was sampled monthly by collection of whole blood from the jugular vein for evaluation of the antibody titre. Further purification of the serum with caprylic acid /ammonium sulphate precipitation was effected.

Example 12: Competitive assay for cross-reactivity measurement

A 96 well ELISA plate was coated with 125μl/well of antibody derived from immunogen 2 (Example 8) at an appropriate concentration prepared in Tris buffer and was incubated at 4°C for overnight. Contents were tipped out and plate washed (x 4) using TBST before 50μl of appropriate cross-reactant was added at various concentrations from 0-10ng/ml. 75μl of tracer (Example 10 - tracer 1) was added to each well at an appropriate dilution, followed by incubation at 25°C for 1 hour. Contents were tipped out and plate washed (x 6) using TBST and 125μl/well TMB solution was added. After 20mins in dark, 125μl/well 0.2M sulphuric acid was added. The plate was read at 450nm using KC junior software. The cross-reactivity (CR) was calculated using the equation below. All calculations were based upon binding and displacement at the 50% of maximum OD (optical density) binding point. The maximum OD is the signal generated using tracer alone and 50% displacement (inhibition) corresponds to the $IC_{50}$. $IC_{50}$ = standard concentration which produces 50% inhibition of maximum signal B/B0 = (B/B0) x 100 %CR = cross-reactivity (OD without cross-reactant - OD with cross-reactant) x 100

*Tables 1a-1c*

Ave OD = average optical density

CV = coefficient of variation of OD

B = absorbance at 450nm at xng/ml standard concentration

$B_0$ = absorbance at 450nm at 0ng/ml standard concentration

$$B/B0 = (B/B0) \times 100$$

$$\%CR = \text{cross-reactivity (OD without cross-reactant - OD with cross-reactant)} \times 100$$

**Results**

Table 1a

| Conc. | MT-45 | | | 3-Hydroxy MT-45 | | |
|---|---|---|---|---|---|---|
| ng/ml | Ave OD | %CV | B/Bo | Ave OD | %CV | B/Bo |
| 0 | 2.098 | 5.8 | 100 | 2.078 | 1.1 | 100 |
| 0.015625 | 1.903 | 3.6 | 91 | 1.905 | 3.3 | 92 |
| 0.03125 | 1.602 | 4.2 | 76 | 1.818 | 1.1 | 87 |
| 0.0625 | 1.506 | 3.2 | 72 | 1.511 | 3.2 | 73 |
| 0.125 | 1.069 | 8 | 51 | 1.203 | 6 | 58 |
| 0.25 | 0.687 | 5.8 | 33 | 0.765 | 7.2 | 37 |
| 0.5 | 0.339 | 4.3 | 16 | 0.389 | 9.3 | 19 |
| 1 | 0.165 | 0.3 | 8 | 0.192 | 9.2 | 9 |
| IC50<br>%CR | 0.131ng/ml<br>100 | | | 0.158ng/ml<br>82.9 | | |

Table 1b

| Conc. ng/ml | MT-45 | | | 3-Hydroxy MT-45 | | |
|---|---|---|---|---|---|---|
| | Ave OD | %CV | B/Bo | Ave OD | %CV | B/Bo |
| 0 | 1.945 | 1.7 | 100 | 1.925 | 1.9 | 100 |
| 0.03125 | 1.594 | 1.6 | 82 | 1.713 | 2.7 | 89 |
| 0.0625 | 1.543 | 2.7 | 79 | 1.573 | 2.2 | 82 |
| 0.125 | 1.286 | 2.9 | 66 | 1.353 | 0.8 | 70 |
| 0.25 | 1.006 | 3.5 | 52 | 1.101 | 1.6 | 57 |
| 0.5 | 0.728 | 4.3 | 37 | 0.811 | 2.3 | 42 |
| 1 | 0.462 | 1.9 | 24 | 0.528 | 0.7 | 27 |
| 2 | 0.276 | 2.8 | 14 | 0.321 | 2.4 | 17 |
| IC50<br>%CR | 0.279ng/ml<br>100 | | | 0.344<br>81.1 | | |

Table 1c

| Conc. ng/ml | MT-45 | | | 3-Hydroxy MT-45 | | |
|---|---|---|---|---|---|---|
| | Ave OD | %CV | B/Bo | Ave OD | %CV | B/Bo |
| 0 | 1.870 | 3.2 | 100 | 1.862 | 6.8 | 100 |
| 0.03125 | 1.549 | 3 | 83 | 1.725 | 1.5 | 93 |
| 0.0625 | 1.523 | 0.5 | 81 | 1.548 | 1.6 | 83 |
| 0.125 | 1.205 | 0.5 | 64 | 1.266 | 3.5 | 68 |
| 0.25 | 0.851 | 1.1 | 46 | 0.927 | 4.4 | 50 |
| 0.5 | 0.527 | 1.3 | 28 | 0.612 | 5 | 33 |
| 1 | 0.294 | 1.4 | 16 | 0.350 | 4 | 19 |
| 2 | 0.155 | 3.9 | 8 | 0.189 | 5 | 10 |
| IC50<br>%CR | 0.221ng/ml<br>100 | | | 0.250ng/ml<br>88.4 | | |

Table 1a - antisera at 0.625μg/ml from sheep 1
Table 1b - antisera at 10.00μg/ml from sheep 2
Table 1c - antisera at 5.00μg/ml from sheep 3

The data of Tables 1-3 (OD and % CV) are average values derived from 3 replicates - the cross-reactivity values for 3-hydroxy MT-45 for sheep 1 and sheep 2 for the three replicates were 75.8%, 80.5% and 92.4% and 73.4%, 82.6% and 87.3%, respectively, compared to MT-45 (100%).

## Claims

**1.** An immunogen of structure

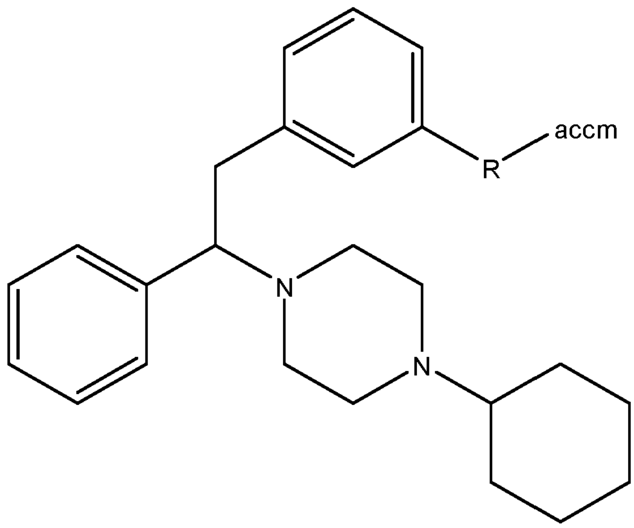

wherein R is a spacing group which is -(Q)m-Y-X-;
m=0 or 1 and Q is -O-, -NH- or -S-;
Y is a $C_{1-6}$ substituted or unsubstituted, straight chain, alkanediyl moiety;
X, before attachment to the antigenicity conferring carrier material, is chosen from carboxy , dithiopyridyl, maleimidyl, amino, hydroxyl, thiol , thioester, or aldehyde;
and R is attached to an antigenicity conferring carrier material (accm) which is keyhole limpet haemocyanin, bovine thyroglobulin, bovine serum albumin, egg ovalbumin, bovine gamma globulin or cationised bovine serum albumin.

**2.** An antibody raised from an immunogen of claim 1 which binds to MT-45.

**3.** The antibody of claim 2 raised from an immunogen in which Q is -O-, Y is $-CH_2-$, X is carboxy and the accm is keyhole limpet haemocyanin, which has an $IC_{50}$ of <1.00 ng/ml to MT-45 as calculated from a standard curve using MT-45-CME-HCTL-maleimide-HRP (Tracer 2) as detecting agent.

**4.** An immunoassay method of detecting or determining MT-45 in an *in vitro* sample or in a solution comprising contacting the sample or solution with a detecting agent and an antibody of claims 2 or 3 and detecting the bound detecting agent in which the detecting agent is

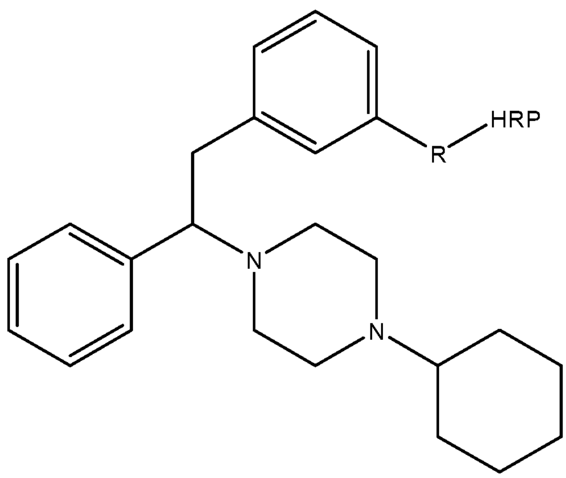

in which R of the detecting agent is a crosslinking group and HRP is horseradish peroxidase, and deducing the presence or amount of MT-45.

**5.** A detecting agent of structure

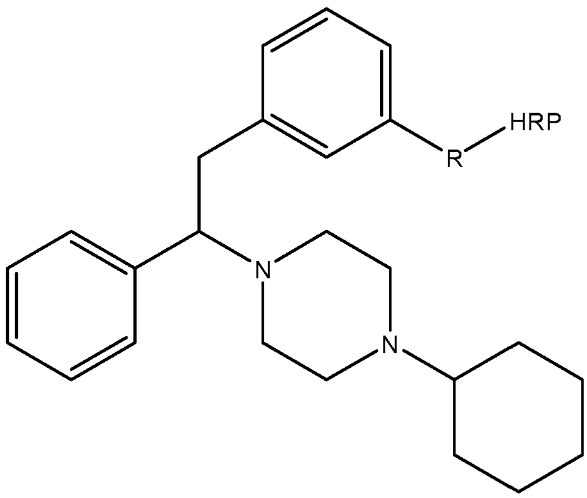

in which R of the detecting agent is a crosslinking group and HRP is horseradish peroxidase.

**6.** The detecting agent of claim 5 which has the structure

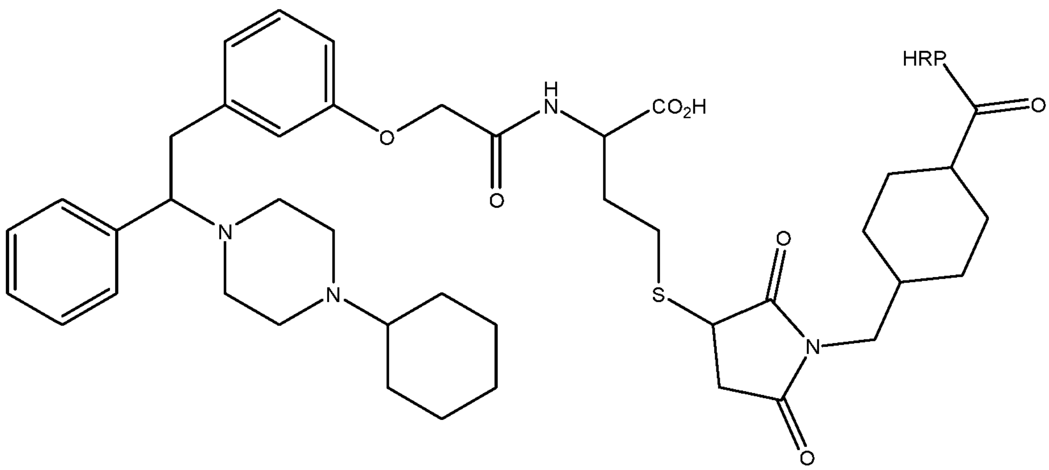

**7.** A kit comprising an antibody of either of claims 2 or 3 and optionally a detecting agent as described in claim 6.

**Patentansprüche**

**1.** Ein Immunogen der Struktur

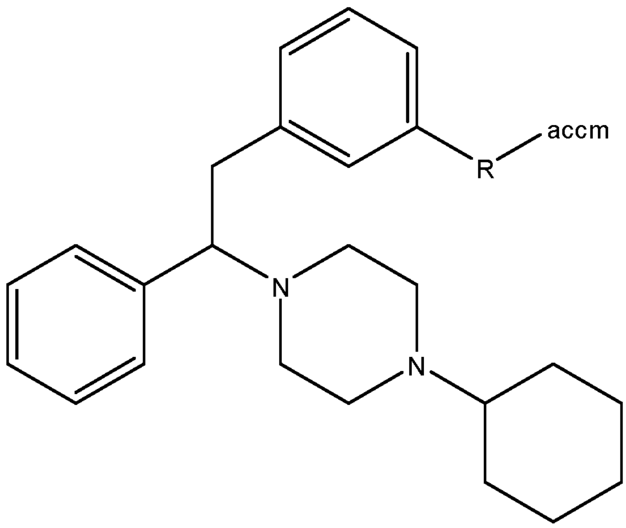

wobei R eine Abstandsgruppe ist, die -(Q)m-Y-X- ist;
m = 0 oder 1 und Q -O-, -NH- oder -S- ist;
Y eine C1-6-substituierte oder unsubstituierte, geradkettige Alkandiylgruppe ist;
X, vor der Anbindung an das Antigenität verleihende Trägermaterial, ausgewählt ist aus Carboxy, Dithiopyridyl, Maleimidyl, Amino, Hydroxy, Thiol, Thioester oder Aldehyd;
und R an ein Antigenität verleihendes Trägermaterial (ACCM) gebunden ist, das Keyhole-Limpet-Hämocyanin, Rinderthyreoglobulin, Rinderserumalbumin, Ei-Ovalbumin, Rindergammaglobulin oder kationisiertes Rinderserumalbumin ist.

**2.** Ein Antikörper, erzeugt aus einem Immunogen nach Anspruch 1, der an MT-45 bindet.

**3.** Der Antikörper nach Anspruch 2, erzeugt aus einem Immunogen, in dem Q für -O-, Y für $-CH_2-$, X für Carboxy und accm für Keyhole-Limpet-Hämocyanin steht, weist einen $IC_{50}$-Wert von <1,00 ng/ml für MT-45 auf, berechnet aus einer Standardkurve unter Verwendung von MT-45-CME-HCTL-Maleimid-HRP (Tracer 2) als Nachweisreagenz.

**4.** Ein Immunoassay-Verfahren zum Nachweis oder zur Bestimmung von MT-45 in einer In-vitro-Probe oder in einer Lösung, umfassend das Kontaktieren der Probe oder Lösung mit einem Nachweisreagenz und einem Antikörper nach Anspruch 2 oder 3 und den Nachweis des gebundenen Nachweisreagenz, wobei das Nachweisreagenz

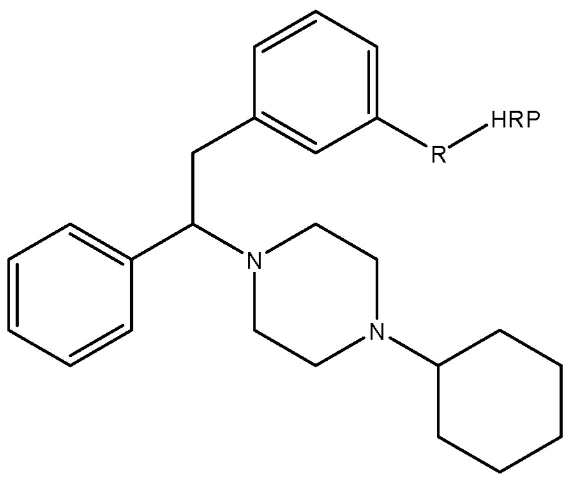

wobei R des Detektionsmittels eine Vernetzungsgruppe und HRP Meerrettichperoxidase ist, und die Bestimmung des Vorhandenseins oder der Menge von MT-45.

**5.** Ein Detektionsmittel der Struktur

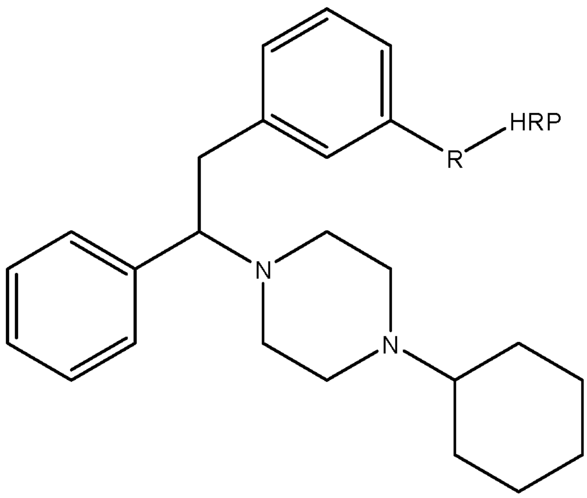

wobei R des Detektionsmittels eine Vernetzungsgruppe und HRP Meerrettichperoxidase ist.

6. Das Detektionsmittel nach Anspruch 5 mit der Struktur

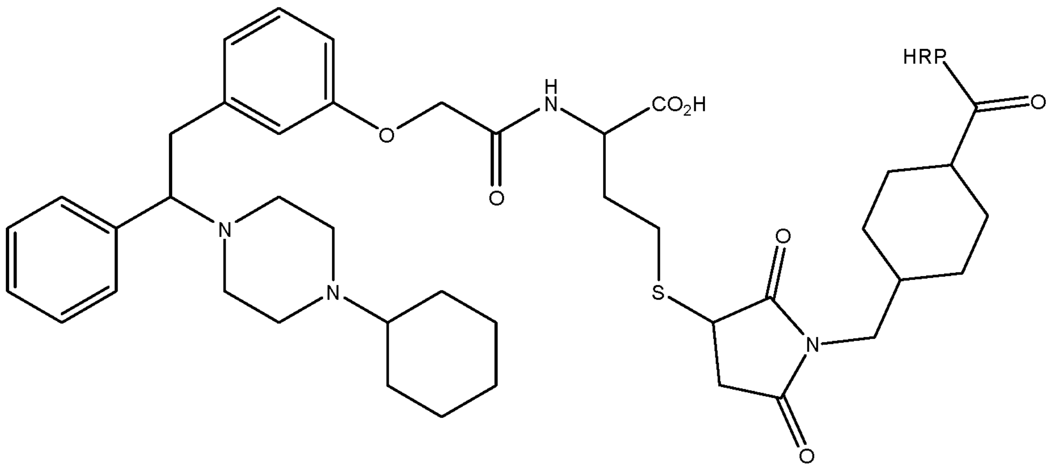

7. Ein Kit, umfassend einen Antikörper gemäß Anspruch 2 oder 3 und optional ein Nachweismittel wie in Anspruch 6 beschrieben.

**Revendications**

1. Un immunogène de structure

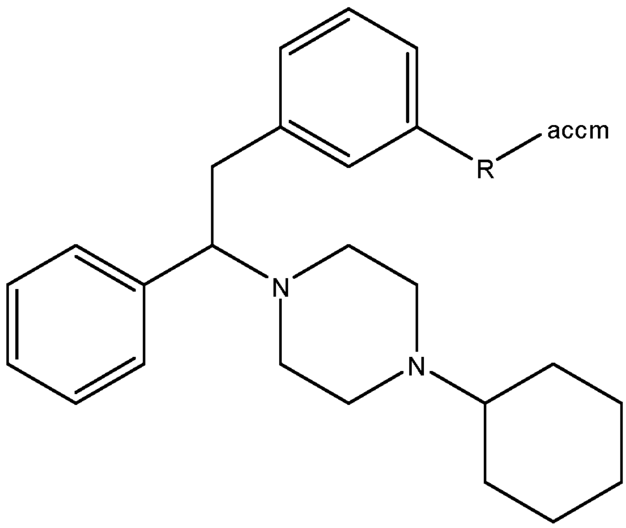

où R est un groupe d'espacement -(Q)m-Y-X- ;
m = 0 ou 1 et Q est -O-, -NH- ou -S- ;
Y est un groupe alcanediyle à chaîne droite, substitué ou non en C1-6 ;
X, avant fixation au support conférant l'antigénicité, est choisi parmi les groupes carboxy, dithiopyridyle, maléimidyle, amino, hydroxyle, thiol, thioester ou aldéhyde ; et R est fixé à un support conférant l'antigénicité (accm) qui est l'hémocyanine de patelle, la thyroglobuline bovine, l'albumine sérique bovine, l'ovalbumine d'œuf, la gammaglobuline bovine ou l'albumine sérique bovine cationisée.

**2.** Anticorps obtenu à partir d'un immunogène selon la revendication 1, qui se lie au MT-45.

**3.** Anticorps selon la revendication 2, obtenu à partir d'un immunogène dans lequel Q représente -O-, Y représente -CH2-, X représente un groupe carboxyle et l'anticorps est l'hémocyanine de patelle, dont la CI50 pour le MT-45 est inférieure à 1,00 ng/ml, calculée à partir d'une courbe d'étalonnage utilisant le MT-45-CME-HCTL-maléimide-HRP (traceur 2) comme agent de détection.

**4.** Méthode d'immunoessai pour la détection ou la détermination du MT-45 dans un échantillon in vitro ou dans une solution, comprenant la mise en contact de l'échantillon ou de la solution avec un agent de détection et un anticorps selon les revendications 2 ou 3, et la détection de l'agent de détection lié, l'agent de détection étant

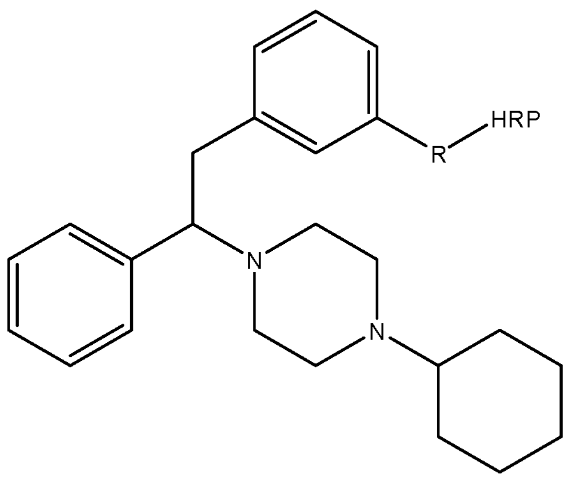

où R de l'agent de détection est un groupe de réticulation et HRP est la peroxydase de raifort, et déduire la présence ou la quantité de MT-45.

**5.** Agent de détection de structure

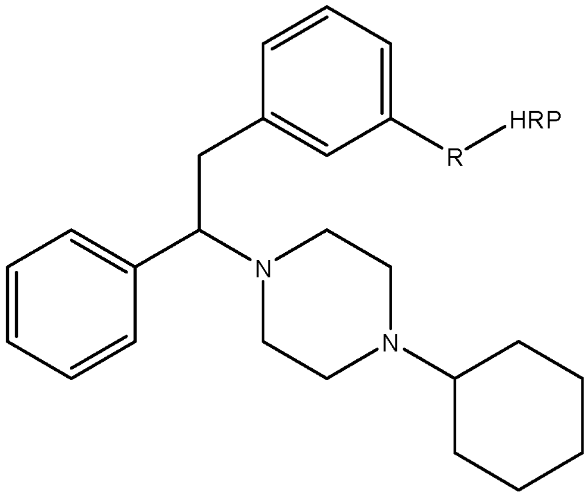

dans laquelle R de l'agent de détection est un groupe de réticulation et HRP est la peroxydase de raifort.

**6.** Agent de détection selon la revendication 5, dont la structure est la suivante :

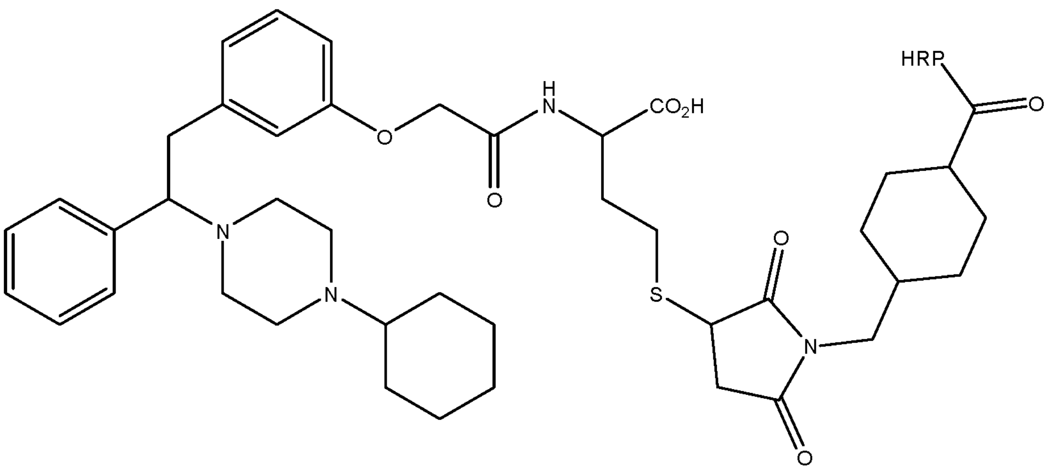

**7.** Kit comprenant un anticorps selon l'une quelconque des revendications 2 ou 3 et éventuellement un agent de détection tel que décrit dans la revendication 6.

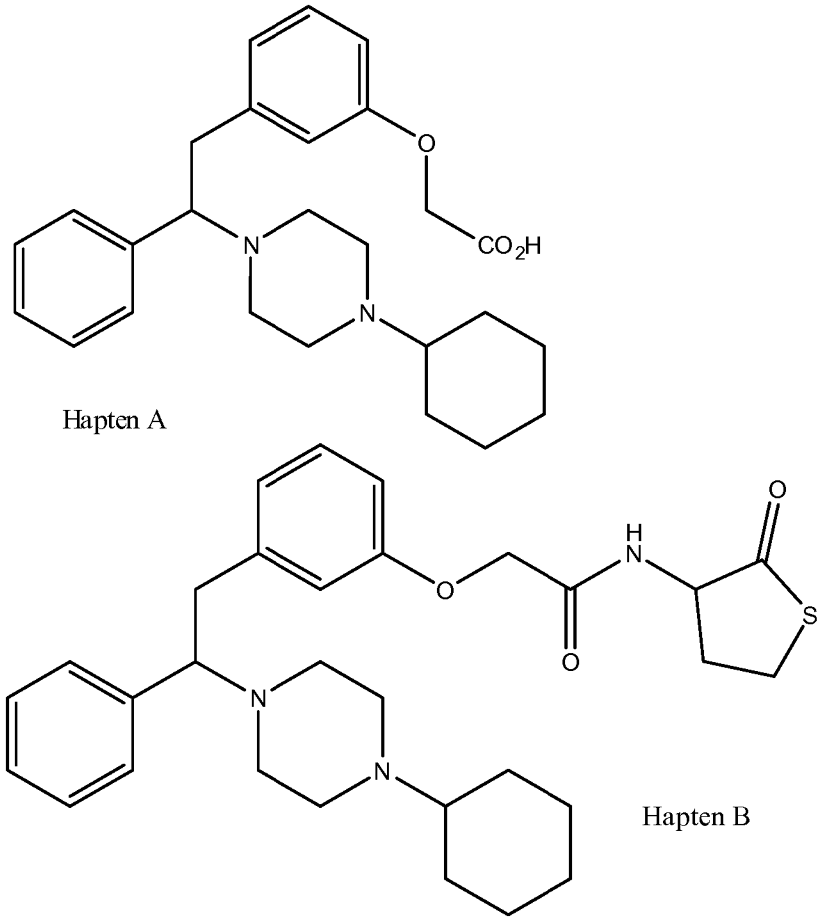

Hapten A

Hapten B

**Figure 1**

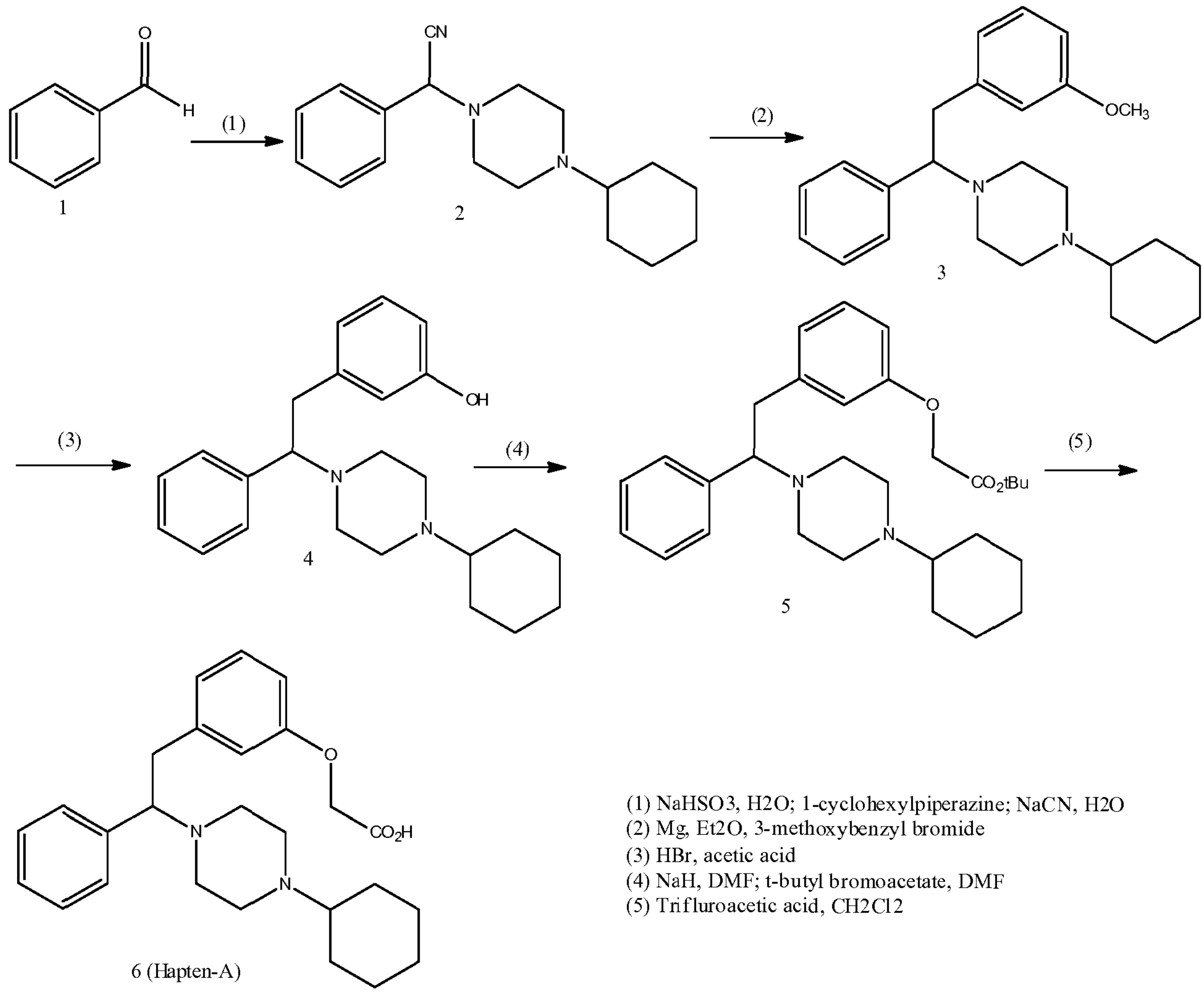
1
(1)
2
(2)
3
(3)
4
(4)
5
(5)
6 (Hapten-A)
(1) NaHSO3, H2O; 1-cyclohexylpiperazine; NaCN, H2O
(2) Mg, Et2O, 3-methoxybenzyl bromide
(3) HBr, acetic acid
(4) NaH, DMF; t-butyl bromoacetate, DMF
(5) Trifluroacetic acid, CH2Cl2

**Figure 2**

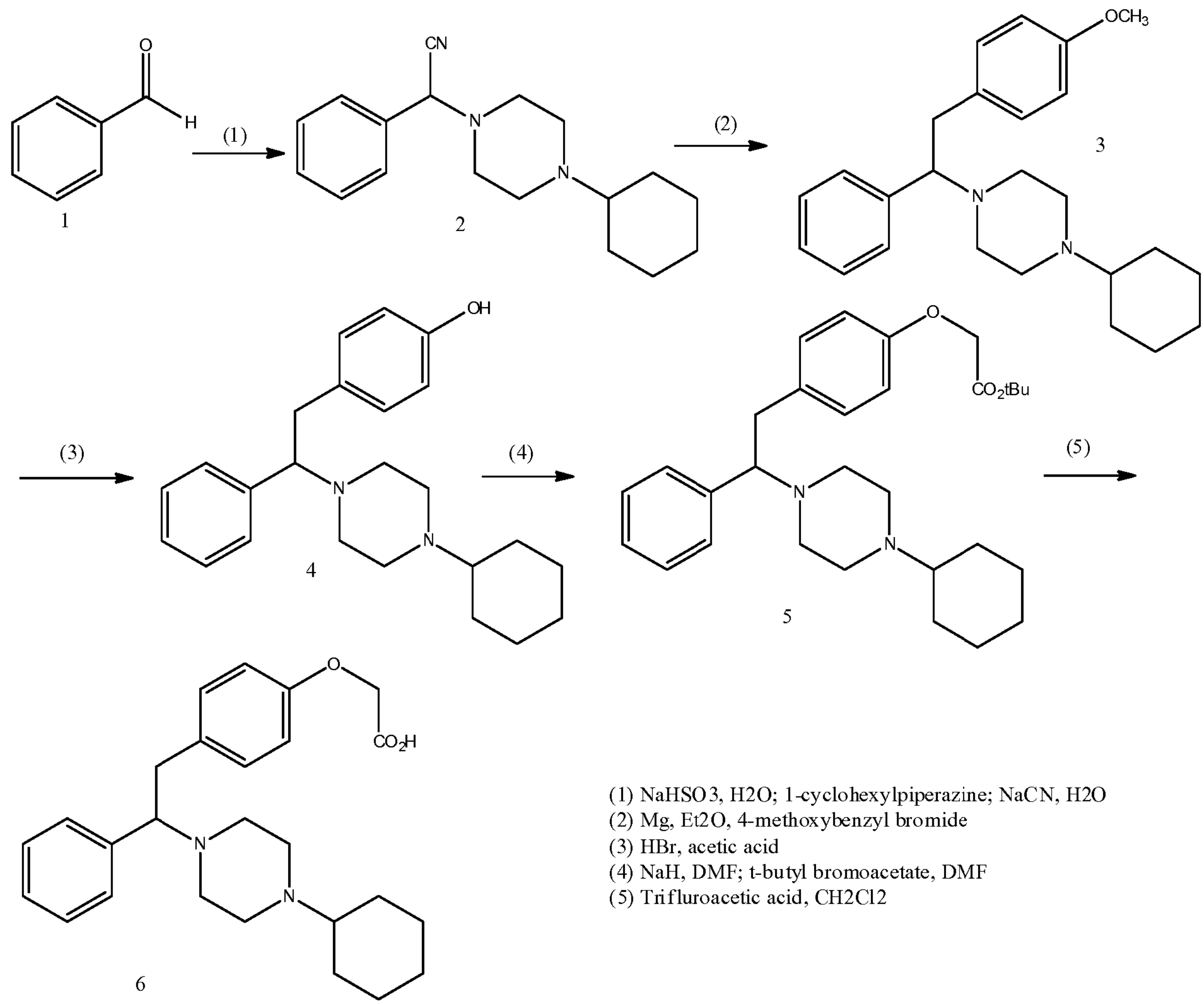
(1)
(2)
(3)
(4)
(5)
1
2
3
4
5
6
(1) NaHSO3, H2O; 1-cyclohexylpiperazine; NaCN, H2O
(2) Mg, Et2O, 4-methoxybenzyl bromide
(3) HBr, acetic acid
(4) NaH, DMF; t-butyl bromoacetate, DMF
(5) Trifluroacetic acid, CH2Cl2

**Figure 3**

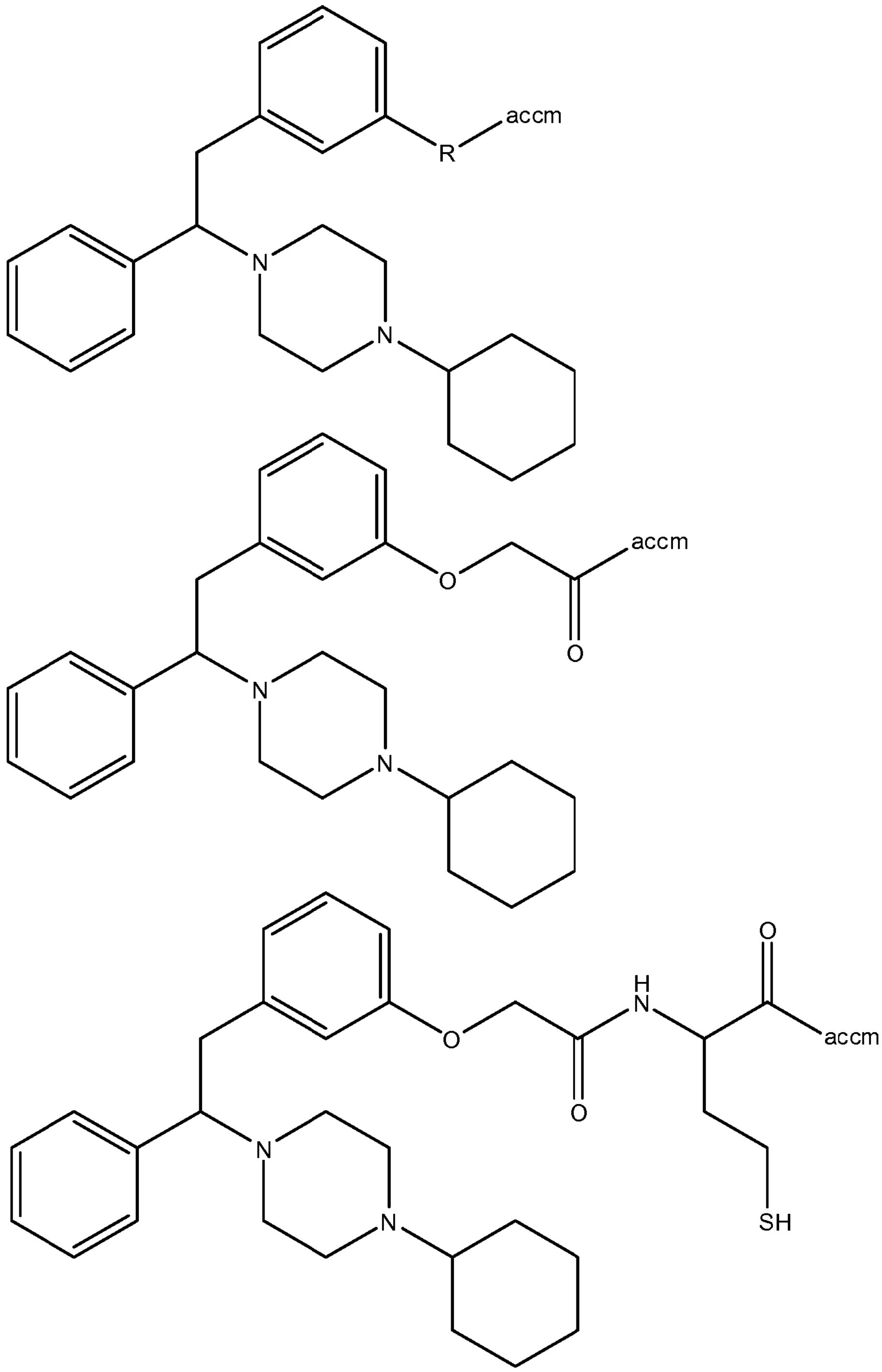

accm = antigenicty conferring carrier material

**Figure 4**

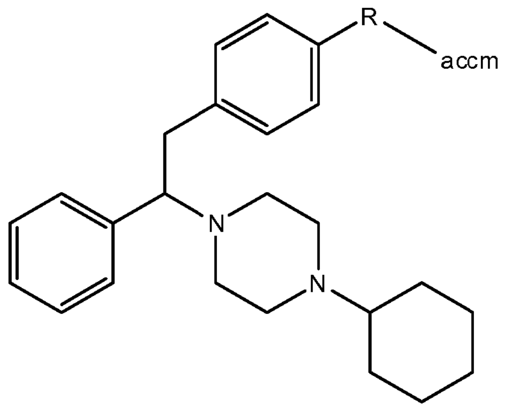

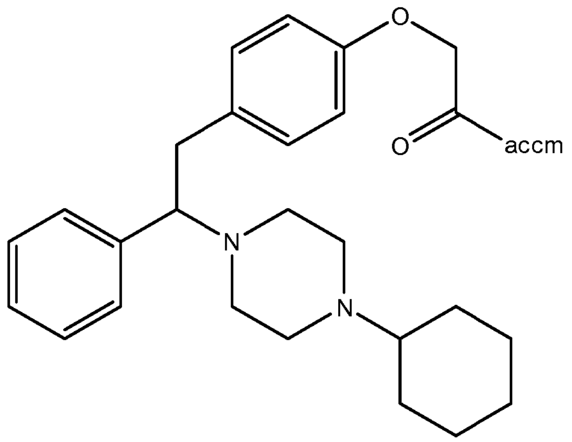

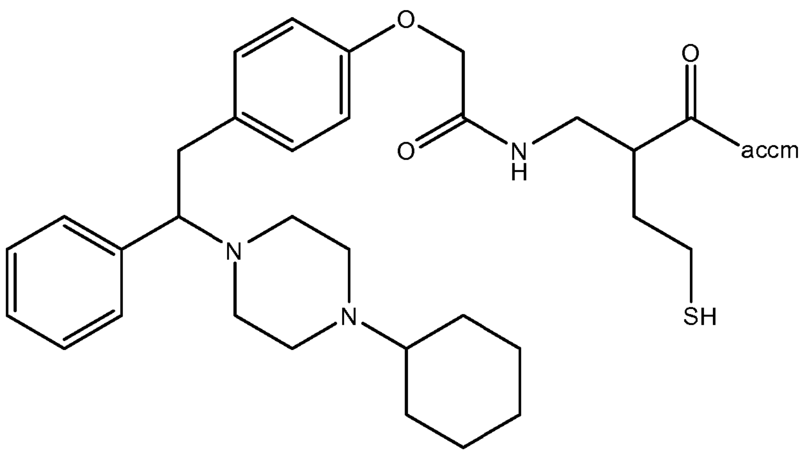

accm= antigenicty conferring carrier material

**Figure 5**

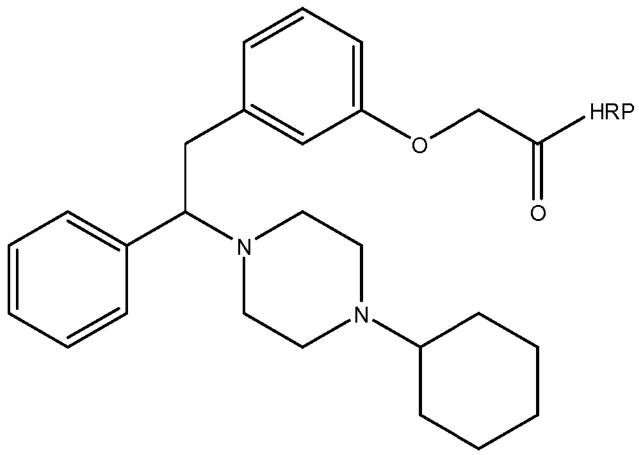

Tracer 1

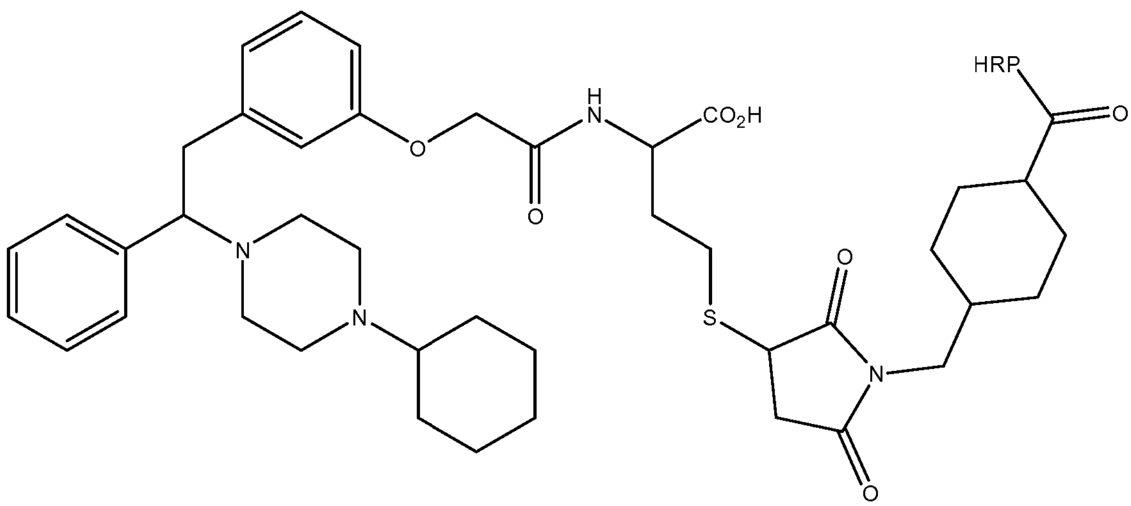

Tracer 2

**Figure 6**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- EMCDDA-Europol Joint Report on a new psychoactive substance: 1-cyclohexyl-4-(1,2-diphenylethyl) piperazine ('MT-45'). Joint Reports. Publications Office of the European Union, 2014
- *Risk Assessment Report of a new psychoactive substance: 1-cyclohexyl-4-(1,2-diphenylethyl)piperazine (MT-45)*, 2014
- **PAPSUN et al.** Analysis of MT-45, a novel synthetic opioid, in human whole blood by LC-MS-MS and its identification in a drug-related death.. *Journal of Analytical Toxicology*, 2016, vol. 40, 313-317
- Bioconjugate Techniques. Thermo Scientific Crosslinking Technical Handbook. Academic Press, 1996, vol. 1606073, 785